# EUROPEAN PATENT SPECIFICATION

(11) **EP 3 890 699 B1**
(45) Date of publication and mention of the grant of the patent: **14.01.2026**
(21) Application number: 19821023.9
(22) Date of filing: 28.11.2019
(51) Int. Cl.: A61K 9/00, A61K 47/02, A61K 47/06, A61K 9/06

(54) **HYPERTONIC OPHTHALMIC OINTMENT**
HYPERTONE OPHTHALMISCHE SALBE
ONGUENT OPHTALMIQUE HYPERTONIQUE

(30) Priority: 04.12.2018 IT 201800010796
(43) Date of publication of application: 13.10.2021
(73) Proprietor: Orfeo, Vincenzo, 80122 Napoli (IT)
(72) Inventor: Orfeo, Vincenzo, 80122 Napoli (IT)
(74) Representative: Bianchetti & Minoja with Trevisan & Cuonzo IPS SRL
(86) International application number: PCT/EP2019/082868
(87) International publication number: WO 2020/114875

(56) References cited:
- WO-A1-2011/088745
- WO-A1-2015/150459
- WO-A1-88/09665
- G. HO WANG YIN ET AL: "Acuité visuelle, pachymétrie et densité cornéenne après traitement par solution hyperosmolaire au chlorure de sodium 5 % dans l'oedème cornéen postopératoire", JOURNAL FRANCAIS D'OPHTALMOLOGIE., vol. 38, no. 10, 1 December 2015 (2015-12-01), FR, pages 967 - 973, XP055611051, ISSN: 0181-5512, DOI: 10.1016/j.jfo.2015.07.002

## Description

The present invention provides an ophthalmic ointment comprising an hydrophilic phase dispersed in a lipophilic phase by means of an emulsifier, wherein the hydrophilic phase contains a saturated solution of sodium chloride and hyaluronic acid or salt. The ointment is conveniently used in the treatment of corneal oedema.

### BACKGROUND TO THE INVENTION

Ointments are semisolid systems that usually act as viscoelastic materials when subjected to friction, more correctly described as "shearing stress". They usually contain medicaments and are intended to be applied externally to the body or the mucosa. Many medicaments intended for topical application to intact or broken skin or the mucosa have said semisolid consistency, designated as an ointment, in addition to creams, ointments, pastes etc., which are mainly used as skin protection agents or emollients. Unlike the latter forms, an ointment consists almost entirely of fatty ingredients. An ointment may or may not be medicated.

The base of the ointment is prepared, and the ointment is formulated, by incorporating the active ingredients by crushing/grinding them in the most effective ratio. When the formulation has been completed, the quality of the ointment is evaluated in terms of liability to cause irritation, spreadability, diffusion and stability. Ointments are viscous and applied topically on a variety of body surfaces, such as the skin of the chest, over a joint, or on the mucosa of the eye, vagina, anus or nose. Medicated ointments can contain a medicament dissolved, suspended or emulsified in the base.

Ointments are used topically for various purposes, e.g. as antiseptics, emollients, anti-itching agents, keratolytics, hydrating agents and astringents.

A typical basic formula of an ointment is as follows:
Ingredients (per 100 g)

| | |
|---|---|
| Lanolin | 5.0 g |
| Hard paraffin wax | 5.0 g |
| Cetostearyl alcohol | 5.0 g |
| Soft white or yellow paraffin wax | 85.0 g |

All the ingredients are dissolved hot together and stirred until cold.

If used as an emollient, the ointment incorporates small amounts of aqueous solutions.

The characteristics of the ideal ointment are as follows:
- physically and chemically stable
- the base must have no therapeutic action
- any finely divided active ingredient must be distributed evenly in the ointment base.

Hypertonic ointments for ophthalmic use, i.e. those having a high content of osmotic component, are relevant to the present invention.

Said high content has the therapeutic properties of extracting from the tissues excess of water that causes swelling of membranes in forms of oedema.

The formation of corneal oedema is a separate process from that associated with the accumulation of abnormal amounts of tissue fluids in other parts of the body.

As the cornea is avascular, changes in its state of hydration are not correlated with changes in vascular permeability, which is the common cause of oedema. Corneal oedema can be found in a variety of clinical situations. Although it may have different aetiologies, the physiopathological mechanisms that give rise to corneal oedema originate from mechanical and/or chemical damage to the corneal endothelium. The latter is a single cell layer along the inner surface of the cornea that continuously pumps fluid from the cornea, keeping the cornea free. When said cells are damaged, they cannot regenerate, and fluid accumulates in the cornea, leading to swelling (oedema) of the cornea and gradual blurring of the vision.

There are numerous hypertonic products, medicaments and medical devices on the market containing osmotic agents designed to treat corneal oedema.

The best-known hypertonic ophthalmic ointment on the market is a product for corneal oedema manufactured by Bausch & Lomb, namely MURO 128^{®}, which contains lanolin, mineral oil, white vaseline and water in addition to 5% sodium chloride. The same reference composition is present in the USP generic ophthalmic ointment, Sochlor^{®} (sodium chloride) and Visinic^{®}, which also contain 5% sodium chloride.

The most common medical devices include eyedrops and ointments containing salts such as sodium chloride or polyalcohols such as mannitol. Eyedrops are often recommended for daytime use but require frequent administration in view of their short residence time in the eye because of the leaching action of the lacrimal fluid.

Ointment-based preparations containing an osmotic agent, due to their lipophilic characteristics, provide a longer residence time in the eye than aqueous solutions, and therefore require fewer administrations. However, they present the problem that the osmotic component is segregated from the fatty matrix of the ointment and is not completely accessible to diffusion by the lacrimal fluid, which is hydrophilic. This limits the optimum operation of the ointment.

The most common, simple and effective functional component to induce osmotic attraction of water from the tissues is sodium chloride.

Sodium chloride is a salt which, due to its widespread presence and high compatibility, constitutes the first choice for generating osmotic attraction of water as sodium and chlorine ions are completely dissociated in water.

When the active agent carried by the ointment is hydrophilic, as in the case of sodium chloride, although its residence time is lengthy, its exit from the ointment is often incomplete, because there is no miscibility between the aqueous medium of the lacrimal fluid and the lipophilic ingredients of the ointment. If an hydrophilic component is in the solid state, it could even be postulated that it can never be dissolved or extracted from the interior of a hydrophobic fatty matrix such as that of an ointment in order to act where required in the eye. This is the typical case of sodium chloride, having the function of osmotic agent.

When a hypertonic ophthalmic product is used, one of the most strongly felt problems is the stinging caused by the attraction of water from the tissues generated by the high concentration of osmotic agent.

Due to the hydrating and tissue collagen-reconstituting properties of hyaluronic acid, it has been observed in some recent studies (G. Ho Wang Yin et al., Journal Francais d'Ophtalmologie, vol. 38 no. 10; 2015) that the addition of said polymer to an osmotic saline solution attenuates the intensity of the stinging.

Hyaluronic acid, a polymer of N-acetylglucosamine and glucuronic acid, is widely distributed in the skin, connective tissue and synovial fluid. In the eye, hyaluronic acid is found in the vitreous humour, aqueous humour and connective tissues. In particular, dedicated studies demonstrate a clear advantage of hyaluronic acid over saline solution, not only in the subjective evaluation of relief from symptoms and the duration of such relief, but also in objective tests on structure and ophthalmic function.

The average molecular weight of the commercially available hyaluronic acid can range from a low molecular weight (20 kDa) to a high molecular weight (1,500 to 3,000 kDa), via an intermediate grade (800 kDa).

A further problem of an ophthalmic preparation in ointment form is its low transparency compared with aqueous eyedrops, which can limit or even prevent correct vision. This effect often causes aqueous forms to be preferred for daytime use, because although they are more rapidly eliminated from the lacrimal fluid, the aqueous forms are more convenient in daytime, as they do not interfere with a patient's normal requirements (reading, driving and eyesight in general).

At night, or whenever the patient sleeps, the lower transparency of the ointment is not considered to be a drawback, so that its action of prolonging contact with the cornea can be fully exploited, the only remaining problem being, in view of the denser consistency and longer residence time of the ointment in the corneal area, an unpleasant adherence of the two ends of the eyelids to one another, which often prevents spontaneous opening of the eye on waking.

### KNOWN ART

WO2013/043832 discloses hypertonic ophthalmic compositions containing a polysaccharide such as dextran, which are useful in the treatment of recurrent corneal lesions.

Hypertonic ophthalmic compositions containing hyaluronic acid or an acceptable salt thereof are described in US 2017/0128484 Al.

WO2011/088745 discloses an ophthalmic topical anti-bacterial composition which is in the form of an eye ointment containing, besides the antibiotic linezolid, anhydrous lanolin, liquid paraffin and vaseline. The composition may additionally contain sodium hyaluronate as a thickener. There is no mention of eye ointments containing a hydrophilic phase.

G. Ho Wang Yin et al., Journal Francais d'Ophtalmologie, vol. 38 no. 10 (1/12/2015) disclose a hypertonic solution containing sodium chloride 5% associated with sodium hyaluronate 0.15%, and the beneficial effects thereof on corneal oedema.

WO2015/150459 discloses a hypertonic composition, particularly in the form of a solution, containing sodium chloride as the osmotic agent and sodium hyaluronate, and the use thereof for the treatment of corneal oedema.

WO88/09665A1 discloses high salt concentration ophthalmic ointments. For example, a hypertonic ointment of sodium chloride 5% in a base of mineral oil, lanolin, white petrolatum and water is marketed by Muro Pharmaceuticals, Inc. A similar sodium chloride 5% ointment is also produced by Akorn, Inc. This high level of salt in these ointments is used to provide temporary relief of corneal swelling or edema by dehydrating the tissue.

### DESCRIPTION OF THE INVENTION

The object of the present invention is a hypertonic ophthalmic ointment comprising:
- a lipophilic phase consisting of white vaseline and liquid paraffin;
- an hydrophilic phase consisting of water saturated with sodium chloride and containing hyaluronic acid or a pharmaceutically acceptable salt thereof, and wherein the hydrophilic phase further contains a pH regulator which is a phosphate buffer;
- an emulsifying agent which is based on lanolin,
for use in the treatment of corneal oedema.

Unlike a conventional ointment, which is totally lipophilic and therefore water-repellent, the formulation according to the invention allows more complete diffusion of the saturated solution of sodium chloride into the surrounding tissues. It also solves the technical problem of undesirable eyelid sticking associated with pharmacological treatment with ointments, offering considerable therapeutic advantages, while maintaining the already known characteristics of ointments, namely high and prolonged residence.

The hyaluronic acid used, or the pharmaceutically acceptable salt thereof, is preferably the low-molecular-weight type, having an average molecular weight ranging between 20 kDa and 200 kDa, and even more preferably between 30 kDa and 80 kDa.

The pharmaceutically acceptable salt of hyaluronic acid is preferably sodium hyaluronate.

The lipophilic phase of the ointment consists of white vaseline as solid fatty base and liquid paraffin as liquid fatty base.

The emulsifying agent is lanolin-based, such as the product known commercially as Super Hartolan^{™} HC. However, other emulsifying agents, such as sorbitan esters, monoglycerides and fatty alcohols, can also be used.

A pH adjuster, when present, maintains the pH of the composition at a value ranging between 7.0 and 7.4. The pH adjuster is sodium phosphate.

The ointment according to the invention has preferably the following composition.

| **Ingredient** | **Quantity % (g/100 ml)** |
|---|---|
| Sodium chloride | 4.50 - 12.00 |
| Sodium phosphate | 0 - 0.40 |
| Sodium hyaluronate | 0.10 - 1.00 |
| Lipophilic phase | 48.00 - 73.00 |
| Emulsifying agent | 0.20 - 18.00 |
| Water for injection | 14.00 - 34.00 |

In one embodiment of the invention, the ointment has the following composition:

| **Ingredient** | **Quantity % (g/100 ml)** |
|---|---|
| Sodium chloride | 4.50 |
| Sodium phosphate | 0.05 |
| Sodium hyaluronate | 0.40 |
| Lanolin | 8.10 |
| Lipophilic phase | 72.80 |
| Water for injection | 14.15 |

In another embodiment of the invention, the ointment has the following composition:

| **Ingredient** | **Quantity % (g/100 ml)** |
|---|---|
| Sodium chloride | 4.75 |
| Sodium hyaluronate | 0.40 |
| Lanolin | 8.10 |
| White vaseline | 65.08 |
| Liquid paraffin | 6.80 |
| Water for injection | 14.15 |

The ointment according to the invention meets the specifications set out in the table 1 below:

**Table 1**

| Test | Specification limits | |
|---|---|---|
| | MIN | MAX |
| Appearance | *A homogeneous white or pale yellow ointment* | |
| Appearance under the microscope after centrifugation | *No separation should be observed after centrifugation at 3,000 rpm, for 10 min* | |
| Filling weight (g) | ≥ 5 *g* | |
| Melting point (°C) | | *43* |
| Viscosity after sterilisation (cPs) | *150,000* | *250,000* |
| Sterility (for nasal and ophthalmic forms only) | *Sterile* | |

The ointment according to the invention can be prepared by a procedure involving:
- hot preparation of an aqueous phase containing sodium chloride, sodium hyaluronate and optionally sodium phosphate;
- separately, hot preparation in a turboemulsifier of a phase containing the fats and the emulsifying agent;
- addition of the saline aqueous phase to the phase containing the fats and the emulsifying agent, under stirring;
- a first cooling stage under stirring to a temperature of about +50°C;
- a second cooling stage at 35-40°C followed by final homogenisation.

In an experimental model that evaluates the propensity of ointments to cause the eyelids to stick together after ophthalmic application thereof overnight, it was observed that the ointment according to the invention containing sodium hyaluronate enables the eyelids to open more easily than a conventional ointment, while retaining the viscosity characteristics and consequently the residence time of the latter.

The invention is illustrated by the following examples.

### EXAMPLES

It has to be noted that 1000 cPs corresponds to 1 Pa.s.

### Example 1 - Preparation of ointment

- Equipment: melter, turboemulsifier, vessels, spatulas and connecting tubes.
- Preparation of phase A.

Dissolve the salts in demineralised water.

Check that the pH of phase A is between 7.00 and 7.40, and heat the aqueous phase to T=+55°C.

### - Preparation of phase B.

Simultaneously place the paraffin waxes (for example white vaselin and liquid paraffin) and lanolin at T=+70-75°C in the turboemulsifier, under slow stirring, until the phase is transparent.

### - Preparation of final ointment.

Slowly aspirate phase A into phase B under stirring, under vacuum.

When the addition has been completed, continue mixing for 15-20 minutes.

Begin slow cooling with slow planetary stirring.

At T=+50°C, activate the turboemulsifier for 5-10 minutes with "medium" turbo and "fast" planetary stirring.

Continue cooling to a temperature of T=+35-40°C.

Before packaging, homogenise for 5-10 minutes.

### Example 2 - Characterisation of ointment

Physical and functional characterisation tests were conducted on the ointment according to the invention by comparison with a hypertonic ointment already present on the market, also containing sodium chloride, using a reduced-scale skull/eye/eyelid model to evaluate the tendency for the eyelids to stick after 8 hours at external body temperature.

It was observed that the presence of hyaluronic acid in the ointments according to the invention makes it easier for the eyelids to open, without any variations in viscosity, and therefore residence time, compared with a conventional ointment, thereby eliminating the problem that arises on waking the morning after application of a conventional ointment.

### Purpose of the test

The purpose of the test is to characterise the mechanical properties by measuring the force required to open and move the eyelids after administration of the product, by comparing a composition of a hypertonic ointment already present on the market, having a saturated hydrophilic component (i.e. without free water) of sodium chloride, with an ointment according to the invention also containing 0.4% sodium hyaluronate in the saturated hydrophilic component of sodium chloride.

The qualitative/quantitative compositions of the ingredients tested are set out in the comparative table 2 below:

**Table 2**

| **Comparative composition** | | |
|---|---|---|
| | **Hypertonic ointment (present on the market)** | **Hypertonic ointment according to the invention** |
| **Ingredient** | **%** | |
| Sodium chloride | 5.0 | 4.50 |
| Sodium hyaluronate | none | 0.40 |
| Sodium phosphate | none | 0.05 |
| Lanolin | 14.0 | 8.10 |
| Paraffin waxes | 66.6 | 72.80 |
| Water for injection | 14.4 | 14.15 |

### Materials and methods

A reduced-scale physicoanatomical model of the skull with eye and eyelid was created and used to simulate a skull, eyeballs and eyelids, and measure the physical parameters of the performance of the ointment.

The method involves measuring the opening force of the eyelids of the model under different conditions:
- with a common hypertonic ointment present on the market
- with the hypertonic ointment according to the present invention.

Simulated isotonic lacrimal fluid, having the following composition in 100 g:
- sodium chloride 0.670 g
- sodium bicarbonate 0.200 g
- calcium chloride 0.008 g
- magnesium chloride 0.005 g
- potassium chloride 0.138 g
- purified water 98.979 g
is previously deposited on the model of an open eyeball.

### Description of test

The measurements recorded the initial force at t₀ (t = 1 second) and after 480 min (simulating an average period of sleep) required to open/move the eyelids under thermostating conditions at +34°C.

The tests on the samples were conducted in duplicate or triplicate, starting with different tubes of the ointment according to the invention and of the comparator product.

### Operational procedure:

- apply an equal amount of ointment to the edge of the lower eyelid
- close the eyelids
- wait for the time specified above
- measure the strength required to open the eyelids.

### Results and discussion

The results, set out below, show the difference in eyelid opening force (tensile strength) after application of the ointments. Eyelid opening proved sensitive to the presence of the hydrophilic component of the present invention, wherein a lower opening force was recorded than for a classic hypertonic ointment without sodium hyaluronate, and remained practically unchanged after 8 hours.

The system was calibrated with lacrimal fluid on 12 samples treated with artificial tears, obtaining a mean tensile strength of 0.9 N at t=1s (s.d. = 0.12).

The results for the hypertonic ointment without sodium hyaluronate (reference product) over a 8h period are set out below:

| *Hypertonic ointment without* sodium *hyaluronate* | *Tensile Strength [N]* | |
|---|---|---|
| | | |
| | *1 [s]* (t₀) | *8 [h]* |
| Mean of 12 samples | 0.9 | 2.0 |
| S.D. | 0.11 | 0.15 |
| | | |
| | | |
| *Variations [%]* | *Δ₁₋₈ = +55* | |

An increase in eyelid opening force from 0.9 N to 2 N was observed after 8 hours. This increase is probably due to the gradual consolidation of the ointment with the eyelids, and indicates their adherence.

Three tubes of the product according to the invention were tested in the same way, with the following results:

| *Hypertonic ointment according to the invention* | *Tensile Strength [N]* | |
|---|---|---|
| | | |
| | *1 [s]* (t₀) | *8 [h]* |
| Mean of 12 samples | 0.9 | 1.6 |
| S.D. | 0.13 | 0.22 |
| | | |
| | | |
| | | |
| *Variations [%]* | *Δ₁₋₈ = 44* | |

After 8 hours, the opening force was increased to a lesser extent compared to the hypertonic ointment without sodium hyaluronate, demonstrating that the eyelids sticking was substantially reduced (44 vs. 55 % variation of tensile strength) with the invention ointment.

### Example 3

Surgically-treated cataract patients were enrolled in a study for determining the post-surgery corneal oedema with or without administration of the invention ointment.

The oedema observed at day 1 from the cataract surgical treatment was still present after 7 days:

| **Oedema thickness (mm) - mean of 5 patients** | | | |
|---|---|---|---|
| **Before surgery** | **After surgery** | | |
| | Day 1 | Day 3 | Day 7 |
| 579 (s.d. 7) | 611 (s.d. 35) | 604 (s.d. 35) | 592 (s.d. 17) |

Oedema thickness increase after 3 days: 25 micron
Oedema thickness increase after 7 days: 13 micron
The observed reduction is indicative of the natural process of corneal repair.

The oedema recovery rate was significantly higher by applying the ointment of the invention after surgery:

| **Oedema thickness (mm) - mean of 6 patients** | | | |
|---|---|---|---|
| **Before surgery** | **After surgery** | | |
| | Day 1 | Day 3 | Day 7 |
| 550 (s.d. 10) | 611 (s.d. 28) | 569 (s.d. 28) | 551 (s.d. 14) |

Oedema thickness increase after 3 days: 19 micron
Oedema thickness increase after 7 days: 1 micron

### Conclusions

During the product characterisation tests conducted on a physical model of the eye and eyelid, it was observed that when a hydrophilic phase containing sodium hyaluronate was present in the ointment, after 8 hours' continuous contact (simulation of a sleeping period) at the ophthalmic temperature of +34°C, the eyelid opening force was significantly lower compared to that of a common ointment (without hyaluronate dispersed in a hydrophilic phase), enabling the eyelids to be opened more easily by the user after a time corresponding to the sleeping period.

Furthermore, the ointment of invention proved effective in treating corneal oedema caused by cataract surgery.

## Claims

1. Hypertonic ophthalmic ointment comprising:
- a lipophilic phase consisting of white vaseline and liquid paraffin;
- an hydrophilic phase consisting of water saturated with sodium chloride and containing hyaluronic acid or a pharmaceutically acceptable salt thereof, and wherein the hydrophilic phase further contains a pH regulator which is a phosphate buffer;
- an emulsifying agent which is based on lanolin,
for use in the treatment of corneal oedema.

2. Ointment according to claim 1, wherein hyaluronic acid, or its pharmaceutically acceptable salt, has an average molecular weight comprised between 20 kDa and 100 kDa.

3. Ointment according to claim 2, wherein the pharmaceutically acceptable salt of hyaluronic acid is sodium hyaluronate.

4. Ointment according to claims 1-3, having the following composition:
| **Component** | **Amount % (g/100 ml)** |
|---|---|
| Sodium chloride | 4.50 - 12.00 |
| Sodium phosphate | 0 - 0.40 |
| Sodium hyaluronate | 0.10 - 1.00 |
| Lipophilic phase | 48.00 - 73.00 |
| Emulsifying agent | 0.20 - 18.00 |
| Water for injection | 14.00 - 34.00 |

5. Ointment according to claims 1-4, having viscosity comprised between 150 Pa.s (150.000 cPs) and 250 Pa.s (250.000 cPs)

6. Ointment according to claims 1-5, for use in the treatment of corneal oedema caused by cataract surgery.

## Patentansprüche

1. Hypertonische ophthalmische Salbe, umfassend:
- eine lipophile Phase, bestehend aus weißer Vaseline und flüssigem Paraffin;
- eine hydrophile Phase, bestehend aus mit Natriumchlorid gesättigtem Wasser und enthaltend Hyaluronsäure oder ein pharmazeutisch annehmbares Salz davon, und wobei die hydrophile Phase des Weiteren einen pH-Regulator enthält, der ein Phosphatpuffer ist;
- einen Emulgator auf der Basis von Lanolin,
zur Verwendung bei der Behandlung von Hornhautödem.

2. Salbe nach Anspruch 1, wobei die Hyaluronsäure oder das pharmazeutisch annehmbare Salz davon ein durchschnittliches Molekulargewicht zwischen 20 kDa und 100 kDa aufweist.

3. Salbe nach Anspruch 2, wobei das pharmazeutisch annehmbare Salz der Hyaluronsäure Natriumhyaluronat ist.

4. Salbe nach den Ansprüchen 1-3 mit der folgenden Zusammensetzung:
| **Komponente** | **Menge % (g/100 ml)** |
|---|---|
| Natriumchlorid | 4,50-12,00 |
| Natriumphosphat | 0-0,40 |
| Natriumhyaluronat | 0,10-1,00 |
| lipophile Phase | 48,00-73,00 |
| Emulgator | 0,20-18,00 |
| Wasser zur Injektion | 14,00-34,00 |

5. Salbe nach den Ansprüchen 1-4, welche eine Viskosität zwischen 150 Pa.s (150.000 cPs) und 250 Pa.s (250.000 cPs) hat.

6. Salbe nach den Ansprüchen 1-5 zur Verwendung bei der Behandlung von Hornhautödem, verursacht durch Katarakt-Chirurgie.

## Revendications

1. Onguent ophtalmique hypertonique comprenant :
- une phase lipophile composée de vaseline blanche et de paraffine liquide ;
- une phase hydrophile constituée d'eau saturée en chlorure de sodium et contenant de l'acide hyaluronique ou un sel pharmaceutiquement acceptable de celui-ci, et dans laquelle la phase hydrophile contient en outre un régulateur de pH qui est un tampon phosphate ;
- un agent émulsifiant à base de lanoline,
pour une utilisation dans le traitement d'un œdème cornéen.

2. Onguent selon la revendication 1, dans lequel l'acide hyaluronique, ou son sel pharmaceutiquement acceptable, présente un poids moléculaire moyen compris entre 20 kDa et 100 kDa.

3. Onguent selon la revendication 2, dans lequel le sel pharmaceutiquement acceptable d'acide hyaluronique est de l'hyaluronate de sodium.

4. Onguent selon les revendications 1-3, présentant la composition suivante :
| **Composant** | **Quantité en % (g/100 ml)** |
|---|---|
| Chlorure de sodium | 4,50 - 12,00 |
| Phosphate de sodium | 0 - 0,40 |
| Hyaluronate de sodium | 0,10 - 1,00 |
| Phase lipophile | 48,00 - 73,00 |
| Agent émulsifiant | 0,20 - 18,00 |
| Eau pour injection | 14,00 - 34,00 |

5. Onguent selon les revendications 1-4, présentant une viscosité comprise entre 150 Pa.s (150 000 cPs) et 250 Pa.s (250 000 cPs).

6. Onguent selon les revendications 1-5, pour une utilisation dans le traitement d'un œdème cornéen causé par la chirurgie de la cataracte.
